# EUROPEAN PATENT APPLICATION

(11) **EP 3 305 404 A1**
(43) Date of publication of application: **11.04.2018**
(21) Application number: 16193048.2
(22) Date of filing: 10.10.2016
(51) Int. Cl.: B01J 23/80, B01J 35/00, B01J 35/02, B01J 35/10, B01J 37/03, B01J 37/08, C07C 29/154, B01J 37/06

(54) **COPPER/ZINC/ALUMINIUM CATALYST FOR THE METHANOL SYNTHESIS PREPARED FROM A BINARY ZINC-ALUMINIUM PRECURSOR SOLUTION**

(71) Applicant: National Petrochemical Company, Tehran (IR)
(72) Inventor: Bahmani, Mohsen, Tehran (IR); Sahebdelfar, Saeed, Tehran (IR)
(74) Representative: Fuchs Patentanwälte Partnerschaft mbB

(57) **Abstract**

The present invention relates to a Cu/Zn/Al based catalyst with high Cu surface area and low Cu/CuO crystallite size, to a method of its production, and to its use for the synthesis of methanol from synthesis gas.

The preparation method comprises the formation of a colloidal Zn-Al network by preparing an aqueous colloidal solution dissolving at least one water soluble or acid soluble Zn(lI) compound in an aqueous solution comprising at least one AI(III) salt prior to simultaneous precipitation with Cu and Zn containing solutions.
It is believed that the network is capable of accomodating Cu/CuO particles thereby reducing sintering effects.

## Description

### Technical Field

The present invention relates to a Cu/Zn/Al based catalyst, to a method of its production, and to its use for the synthesis of methanol from synthesis gas.

### Background of the invention

Methanol is an important chemical which is produced on industrial scale from synthesis gas mixtures (CO/CO₂/H₂) over a Cu/ZnO/Al₂O₃ catalyst. Methanol worldwide production is over 50 million metric tons per year and also its demands and production capacity are continually increasing. It is chiefly used as a solvent or raw material in paint, pharmaceutical and defense industries. It is also of interest as a fuel and potential energy carrier in the future and a bulk chemical for fixation of captured CO₂.

Industrial methanol synthesis catalysts comprise essentially copper, zinc and aluminum mixed oxides which are prepared by co-precipitation to produce a mixture of copper and zinc hydroxycarbonate precipitate. After that, the obtained precursor is exposed to water washing, drying, calcination and shaping. This method creates precursors with a definite stoichiometry capable of transformation into porous and homogeneous aggregates of Cu and ZnO nanoparticles (NPs). Nearly spherical Cu NPs of a size around 10 nm and ZnO NPs arranged in an alternating manner forming porous aggregates with relatively large exposed Cu surface area up to about 40 m²g⁻¹ are produced.

In the ternary Cu/ZnO/Al₂O₃ catalyst for methanol synthesis, CuO is the main active component; ZnO acts both as an electronic and structural promoter exhibiting a major influence on the catalytic activity, while alumina or other refractory oxides mainly increase the long-term stability by acting as structural promoter.

Catalyst preparation methods typically have been optimized and improved by developing preparation methods which decrease the copper crystallite size, increase copper surface area and pore size distribution to enhance catalytic activity and thermal stability. The performance of the catalyst varies significantly with the preparation method. It also should be noted that precipitation conditions such as concentration of initial solutions, changing the ratio of the catalyst components, precipitation temperature, pH of precipitation, addition of promoters and the duration of ageing of the precursor precipitate play a significant role on the performance of the catalyst. Therefore, it is always an object to enhance activity and stability of the catalyst by employing novel preparation methods.

Although ternary Cu/Zn/Al catalysts are the best known catalysts for conversion of carbon oxides and hydrogen to methanol and primary alcohols, the Cu/Zn ratio varies over a wide range in these catalysts, and copper is usually present in excess. In some formulations, zinc is partially substituted with a fourth element from the group of calcium, magnesium and/or manganese metals. Partial substitution of aluminum oxide acting as thermal stabilizer with chromium oxide has been investigated in some inventions, such as DE-A 2056612, DE-A 2302658 and US 4279781. A typical methanol synthesis catalyst is disclosed in EP-A 0125689. The catalyst contains variable molar ratios of Cu/Zn from 2.8 to 3.8 and 12 mol% aluminum oxide, wherein at least 20% of the pore sizes lie between 20 to 75 A and 80% of the pores are larger than 75 A. A similar catalyst appears in JP-A-07008799, which describes the synthesis of catalysts with a wide range of copper/zinc ratio.

Another notable invention for methanol synthesis catalysts is described in
EP-A 0152809 disclosing catalysts prepared from a precursor of (1) copper oxide and zinc oxide, (2) aluminum oxide as thermal stabilizer, and (3) at least one oxide or carbonate of alkali metals. The synthesized catalyst in oxide form comprises 20 to 70 vol% pores in the range of 1.5 to 7.5 nm. Aluminum oxide is used as dispersed colloidal aluminum hydroxide particles in the form of a sol or gel.

US 2005/0080148 A1 deals with the influence of the reduction of the Cu/Zn ratio on the enhancement of catalytic activity and selectivity, especially at temperatures below 250 °C, and also with increasing thermal stability by decreasing the Cu/Zn ratio. The aim is the synthesis of a catalyst with a Cu/Zn ratio below 2.8, preferably between 1.8 to 2.7, and the use of aluminum oxide in the form of aluminum hydroxide sol as thermal stabilizer. The synthesized catalysts exhibit higher activities especially below 250 °C and higher thermal stabilities compared to the catalysts prepared according to EP-A-0125689 due to using alumina hydrosol as thermal stabilizer. The catalysts in oxide form have a BET surface area of 90 to 120 m²/g, a pore volume of 320 to 500 ml/kg and a copper oxide crystallite size of 6 to 7 nm.

In fact, the presence of a hydrosol inhibits the sintering of the reduced copper crystallites. Most probably, due to the increased temperature, the aluminum oxide formed from aluminum hydrosol in the catalyst results in the formation of a pseudo-network over the surface of the catalyst where copper oxide crystallites prior to reduction and copper crystallites after reduction are located, and in this way, it is obtained an "energy sink". Part of the zinc oxide also acts as a thermal stabilizer by contributing in the formation of this pseudo-network. Furthermore, another part of the zinc oxide acts as a trap for sulfur compounds protecting the active phase from these poisons.

Methanol synthesis catalysts often have the problem of lack of stability and initial rapid activity decay due to sintering and growth of the active metal particles. Therefore, most often the activity drops more than 50% in the first two weeks of operation.

By the growing construction of mega-methanol plants with large reactor volumes, the manufacturing of catalysts with higher performance (higher activity, selectivity and stability) for methanol synthesis is well necessary to reduce the reactor volume by using improved formulations.

With gradual rising reaction temperature (to compensate catalyst deactivation) during operation, the by-products formation generally increases and, therefore, the selectivity of the catalyst decreases. On the other hand, some by-products such as methyl ethyl ketone (MEK) are not removable due to azeotrope formation. Therefore, methanol synthesis catalysts with high selectivity are strictly needed.

The purpose of this invention is to solve the problems described above and to provide a catalyst for methanol synthesis which is improved in activity, lifetime and strength as well as a method of its production.

### Summary of the invention

The present invention relates to a method for the preparation of a Cu/Zn/Al catalyst for methanol synthesis comprising the steps of:
(i) Preparing an aqueous colloidal solution by dissolving at least one water soluble or acid soluble Zn(II) compound in an aqueous solution comprising at least one AI(III) salt;
(ii) Preparing an aqueous solution comprising at least one water soluble or acid soluble Cu(II) salt;
(iii) Preparing an aqueous solution comprising at least one water soluble or acid soluble Zn(II) compound;
(iv) Preparing an aqueous solution comprising at least one carbonate salt;
(v) Combining the aqueous solutions of steps (i), (ii), and (iii);
(vi) Combining the aqueous solutions of steps (iv) and (v);
(vii) Precipitating a solid by keeping the solution of step (vi) at a pH value of 6.0 to 8.0 and at a temperature of 35 °C to 70 °C for a period of time of 0.15 to 2 hours;
(viii) Ageing the precipitated solid of step (vii) by keeping the suspension of step (vii) at a pH value of 6.0 to 8.0 and at a temperature of 35 °C to 70 °C for a period of time of 0.15 to 2 hours;
(ix) Filtering the precipitated solid of step (viii);
(x) Optionally washing the filtered solid of step (ix);
(xi) Optionally drying the filtered solid of step (ix) or the washed solid of step (x);
(xii) Calcining the filtered solid of step (ix) or the washed solid of step (x) or the dried solid of step (xi);
(xiii) Optionally tableting the calcined solid of step (xii).

The present invention also relates to the Cu/Zn/Al catalyst thus obtained and to its use for the synthesis of methanol.

The methanol synthesis catalysts of the present invention comprise as essential components copper, zinc and alumina. They exhibit the three characteristics of high activity, high selectivity and longer lifetime by using dyadic zinc-aluminum solutions for their preparation and by optimizing the preparation conditions. Thus, low-cost methanol synthesis catalysts are prepared showing superior performance compared to catalysts that have been described until now.

### Description of the figures

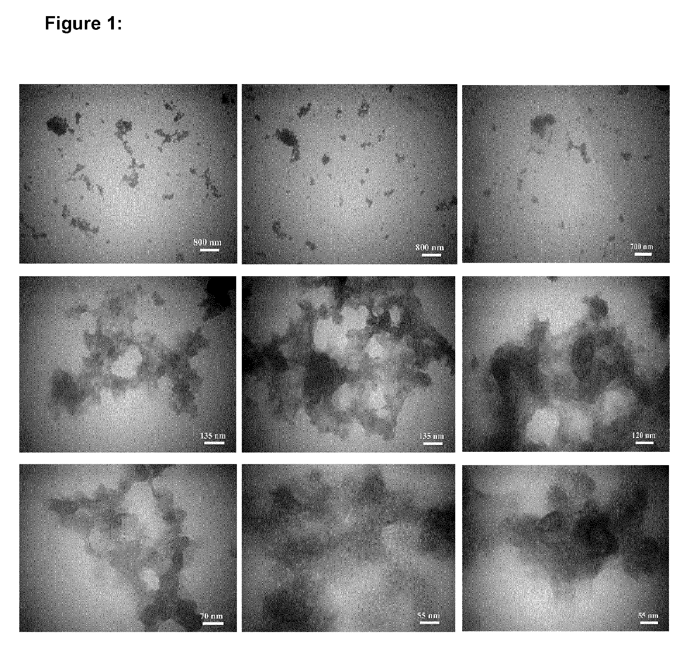

Figure 1 shows the transmission electron microscopy (TEM) image of nanoparticles in the dyadic Al-Zn solution obtained in Example 5 at different localities and magnifications. The Cu particles could be ensnared in the 3D morphology of the Al-Zn network. TEM was performed with a *Zeiss EM10C* microscope operated at 100 kV.

### Detailed description of the invention

In a first aspect, the present invention relates to a method for the preparation of a Cu/Zn/Al catalyst for methanol synthesis comprising the steps of:
(i) Preparing an aqueous colloidal solution by dissolving at least one water soluble or acid soluble Zn(II) compound in an aqueous solution comprising at least one Al(III) salt;
(ii) Preparing an aqueous solution comprising at least one water soluble or acid soluble Cu(II) salt;
(iii) Preparing an aqueous solution comprising at least one water soluble or acid soluble Zn(II) compound;
(iv) Preparing an aqueous solution comprising at least one carbonate salt;
(v) Combining the aqueous solutions of steps (i), (ii), and (iii);
(vi) Combining the aqueous solutions of steps (iv) and (v);
(vii) Precipitating a solid by keeping the solution of step (vi) at a pH value of 6.0 to 8.0 and at a temperature of 35 °C to 70 °C for a period of time of 0.15 to 2 hours;
(viii) Ageing the precipitated solid of step (vii) by keeping the suspension of step (vii) at a pH value of 6.0 to 8.0 and at a temperature of 35 °C to 70 °C for a period of time of 0.15 to 2 hours;
(ix) Filtering the precipitated solid of step (viii);
(x) Optionally washing the filtered solid of step (ix);
(xi) Optionally drying the filtered solid of step (ix) or the washed solid of step (x);
(xii) Calcining the filtered solid of step (ix) or the washed solid of step (x) or the dried solid of step (xi);
(xiii) Optionally tableting the calcined solid of step (xii).

The preparation parameters of ternary Cu/ZnO/Al₂O₃ catalysts strongly influence their catalytic performance in methanol synthesis form synthesis gas (CO/CO₂/H₂ mixtures). The preparation method of the present invention includes co-precipitation of copper-zinc hydroxycarbonates and aluminum hydroxide from mixed nitrate salt solution using sodium carbonate, sodium bicarbonate or other basic compounds as a precipitating agent, followed by ageing, filtration, optionally washing, optionally drying, calcination and optionally shaping, with each step having its distinct effect on the catalytic properties of the product.

Following experiments carried out on the capability of metallic nitrates to dissolve other component(s) of the catalysts, it was found that aluminum nitrate can dissolve bivalent metallic oxides (MO) and carbonates (MCO₃) possibly according to the following reactions:

Al(NO₃)_{3(*aq*)} + MO₍ₛ₎+H₂O → Al(NO₃)(OH)_{2(*aq*)} + M(NO₃)_{2(*aq*)}

Al(NO₃)_{3(*aq*)} + MCO₃₍ₛ₎+2H₂O → Al(NO₃)(OH)_{2(*aq*)} + M(NO₃)_{2(*aq*)} + H₂CO_{3(*aq*)}

According to the amount of bivalent oxide, aluminum nitrate could give part of its nitrate anion to the second metal added. The substitution of nitrate ions with hydroxides provides the possibility of polymerization of aluminum hydroxyl nitrate by elimination of hydroxyl groups as water. The extent of polymerization depends on pH, temperature, concentration of the solution and amount of metallic oxide added. In other words, the higher the amount of metallic oxide added, the higher is the percentage of hydroxyl groups which cause the increase of aluminum hydroxide polymer chain length.

The use of a solution as described above that hereinafter is termed "dyadic metal-aluminum solution" will be discussed in detail below. In principle, the "metal" could be one or more insoluble compounds of 2A, 1 B, 2B, 4B, 6B and 7B group elements of the periodic table. "Dyadic solution" is a novel starting solution which is shown to improve the catalyst performance in terms of activity, selectivity and lifetime. In other words, when the catalyst of the present invention is prepared using a primary dyadic solution it has an activity and stability at least 2.5 times that of commercial catalysts comprised of copper, zinc and aluminum oxides.

The dyadic solution used according to the invention may be prepared by reaction of aluminum nitrate solution preferably with solid ZnO or zinc nitrate. Other zinc compounds such as the carbonate and hydroxide can also be used.

The resulting solution is assumed to contain zinc nitrate and aluminum hydroxynitrates being capable of forming a sol of polymeric aluminum species as evidenced by scattering of a laser beam in the solution. The polymerization of Al species to colloid range particle size prior to precipitation controls their size in the final products resulting in a catalyst with a high durability.

The catalysts of the invention are obtained by co-precipitation of the metals from a solution prepared by mixing e.g. copper nitrate, zinc nitrate and a zinc-aluminum containing alumina sol with a sodium carbonate solution in a well-mixed reactor. The temperature and pH of the precipitation reactor may be controlled with circulator and rate of sodium carbonate addition, respectively. The temperature is within 35 to 70 °C and the pH within 6.0 to 8.0 with a precipitation time of 0.3 to 2 h.

Preferably, the ratio of Zn(II) to Al(III) in the solution of step (i) is 0.2 to 1.3 by mol.

Preferably, the Cu(II) salt of step (ii) is copper nitrate or copper carbonate or a combination thereof.

Preferably, the Zn(II) compound of step (iii) is zinc nitrate or zinc oxide or a combination thereof.

Preferably, the carbonate salt of step (iv) is sodium carbonate or potassium carbonate or a combination thereof.

Preferably, the combined solution of step (v) comprises Cu(II) in an amount of 0.2 to 3.0 mol/l, Zn(II) in an amount of 0.1 to 2.0 mol/l and Al(III) in an amount of 0.1 to 1.0 mol/l.

After precipitation being completed, the suspension may be continuously transferred to a larger well-mixed ageing vessel. In this vessel, the necessary phase transformations occur until the desired phase producing optimum Cu-ZnO contact in the final catalyst is formed as indicated by a color change from blue to blue-green. The ageing is performed at a temperature of 40 to 90°C and at a pH is 6.5 to 8.0, and the aging time is 0.3 to 3 h.

Preferably, steps (vii) and (viii) are performed in two separate reactors having different sizes, wherein the retention times are 0.1 to 1.0 hour in the first reactor and 0.2 to 2.0 hours in the second reactor.

The precipitate is filtered and may be washed with deionized water until the sodium content in the mother liquor falls below 50 ppm. Alkali metals such as sodium increase higher alcohols production. The precipitate may be dried at 110 °C overnight. The sample is then calcined preferably at 300-400 °C under air flow for 4 h to decompose the carbonates and hydroxides of the consistent metals to the corresponding oxides. Lower temperatures could result in incomplete decomposition and higher temperatures could cause sintering of the oxides and reducing surface area of the final catalyst.

The precipitate may then be milled and mixed with a lubricant, preferably graphite up to 2 wt% based on the precipitate, before being formed as pellets in a rotary tablet press.

The weight fractions of copper, zinc and aluminum in the catalyst are preferably from 0.5 to 0.8, from 0.1 to 0.4, and from 0.04 to 0.16, respectively. Copper is the active component. Zinc oxide acts both as textural and chemical promoter while alumina is a textural promoter. Zinc also control the oxidation state of the surface copper present on the catalyst and has considerable effects on catalytic activity. The level of the catalyst components should be kept in the limits for the reasons given below. Since copper is the active component, no sufficient methanol synthesis activity will probably be obtained for loadings below 50 wt%. Contents higher than 80 wt% probably impact copper disparity due to sintering and reduce catalyst stability. Zinc functions to control the oxidation state of surface copper atoms. The optimum interaction occurs in the limits shown. Alumina contents higher than 16 wt% probably increase the acidic sites above the acceptable level which promotes dimethyl ether (DME) formation thereby reducing the selectivity to methanol. Alumina content lower 4 wt% probably is detrimental to catalyst stability and requires addition of other textural promoters such as magnesia or zirconia.

In a further aspect the present invention relates to a Cu/Zn/Al catalyst for methanol synthesis, preferably obtained or obtainable by the method described herein.

The catalysts of the invention have a small particle size and narrow copper particle size as indicated by N₂O titration and nitrogen adsorption/desorption, respectively.

Preferably, the surface area of elemental copper as measured by N₂O chemisorption is at least 40 m²/g catalyst, more preferably at least 45 m²/g catalyst, most preferably at least 55 m²/g catalyst.

Preferably, the copper oxides form particles having a diameter in the range of 2 to 12 nm, more preferably in the range of 3 to 7 nm, as measured by XRD.

Preferably, the elemental copper forms particles having a diameter in the range of 4 to 15 nm, more preferably in the range of 5 to 9 nm, as calculated based on by N₂O chemisorption.

Preferably, the BET surface area as determined by N₂ adsorption is at least 100 m²/g catalyst, more preferably at least 120 m²/g catalyst; the total pore volume as determined by N₂ adsorption at a relative pressure P/Po of 0.98 is at least 0.45 cm³/g catalyst, more preferably at least 0.55 cm³/g catalyst, most preferably at least 0.65 cm³/g catalyst; and the average pore diameter is in the range of 10 to 35 nm, more preferably in the range of 15 to 27 nm.

In still a further aspect the present invention relates to the use of a catalyst as described herein for the synthesis of methanol.

The catalysts of the invention can be used for synthesis of methanol from a CO₂-rich syngas. Before loading, they can be crushed and sieved according to reactor dimensions and feed flow rate. Before introduction of the feed, the catalysts should be activated by a reducing agent such hydrogen or carbon monoxide, preferably hydrogen, using 0.5 to 5.0 % H₂ in N₂ gas mixture. A suitable activation procedure according to TPR results is heating at 3 to 10 °C/min and space velocity of 1000 h⁻¹ and holding for 5 h. Controlled reduction ensures lower contact of the catalyst with steam and high temperatures which could result in active phase sintering.

The reaction set-up may comprise a high pressure steel reactor. The feed components may first be passed through mass flow controllers and premixed in a static mixer. The methanol synthesis reaction in the presence of a catalyst of the present invention should be carried out at a temperature of 150 to 300 °C, preferably 200 to 280 °C, at a pressure of 20 to 100 bar, preferably 40 to 80 bar, and at a space velocity of 4000 to 40000 h⁻¹.

The method of the present invention is described in more details below referring to examples and comparative examples which are intended to illustrate aspects of the invention but not to limit the scope thereof.

The water used according to the present invention is preferably deionized by ionexchange.

The iron content of the precursor salts is preferably below 10 ppm. Iron promotes Fischer-Tropsch synthesis and extensive wax formation.

Deionization of water has been carried out for water used in the following examples and comparative examples.

### Examples and comparative examples

### Example 1

This catalyst was prepared from 1 molar precursor metal solutions and 1.1 molar sodium carbonate precipitating agent solution and dyadic zinc-aluminum solution with Zn/Al=0.5.

300 ml of dyadic zinc-aluminum solution with Zn/Al=0.5 and 1 molar in aluminum nitrate was prepared according to the following procedure:
8.1 g zinc oxide powder and 75.0 g aluminum nitrate nonahydrate crystals were mixed and the resulting mixture was dissolved in 262 g water at 45 to 50 °C and stirred for 30 min. The solution was labeled as solution A.
500 ml 1 M zinc nitrate solution was prepared by dissolving 40.7 g ZnO in 418 g water and 113 g 56% nitric acid (solution B).
1200 ml 1 M copper nitrate solution was prepared by dissolving 132.7 g CuCO₃.Cu(OH)₂ in 995 g water and 270 g 56% nitric acid (solution C).

The three solutions A, B and C were mixed in a magnet stirred vessel and warmed up to 55 °C. The 1.1 molar (15 wt%) sodium carbonate solution was also heated in another vessel to the same temperature. Then, both solutions were poured into the precipitation reactor containing 250 ml demineralized water, with constant stirring, to commence co-precipitation which was performed at a pH of 6.3 to 6.5 and a temperature of 52 to 57 °C.

Two separate well-mixed reactors were used for catalyst preparation. Co-precipitation at constant pH and residence time of 10 min was performed in the first reactor. The second vessel with 5-fold volume compared to the first one was used for ageing until color change of the slurry was observed.

After the first few minutes when the level of the first reactor reached to the desired level (the desired level considered as 1 liter based on the residence time of the suspension in the first reactor which was 10 min and was identical for all catalysts), the resulting suspension was continuously transferred to the second reactor controlled at 55 °C and collected there. At the end of ageing, the temperature of the second reactor was raised to 70 °C and ageing continued until a color change was observed. As soon as a color change observed, the pH was set at 6.9 or 7.0 by the addition of carbonate solution, if necessary, and after 5 min the ageing step was stopped and the resulting precipitate was filtered and washed. Washing was continued until the decant water of filtration of the filter cake (mother liquor) contained less than 50 ppm ions at the last washing step. After filtration, drying was performed in an oven at 105 °C for 12 h. Following drying, calcination was performed with a temperature ramp of 5 °C/min to 320 °C and keeping at this temperature for 120 min. After cooling down, the sample was mixed with 2% graphite and tableted. Finally, the tablets were crushed and the particles within the mesh size 16 to 25 were used for reactor performance tests.

### Example 2

This catalyst was prepared from 1.5 molar precursor metal solutions and 1.65 molar (15%) precipitating agent sodium carbonate solution and dyadic zinc-aluminum solution with Zn/Al=0.5.

300 ml of dyadic zinc-aluminum solution with Zn/Al=0.5 and 1.5 molar in aluminum nitrate was prepared according to the following procedure:
12.2 g zinc oxide powder and 112.6 g aluminum nitrate nonahydrate crystals were mixed and the resulting mixture was dissolved in 253 g water at 45 to 50 °C and stirred for 30 min. The solution was labeled as solution D.
500 ml 1.5 M zinc nitrate solution was prepared by dissolving 61.0 g ZnO in 382 g water and 169.5 g 56% nitric acid (solution E).
1200 ml 1.5 M copper nitrate solution was prepared by dissolving 199 g CuCO₃.Cu(OH)₂ in 944 g water and 405 g 56% nitric acid (solution F).

The three solutions D, E and F were mixed in a magnet stirred vessel and warmed up to 55 °C. The 1.65 molar (15 wt%) sodium carbonate solution was also heated in another vessel to the same temperature. Then, both solutions were poured into the precipitation reactor containing 250 ml demineralized water, with constant stirring, to commence co-precipitation which was performed at a pH of 6.3 to 6.5 and a temperature of 52 to 57 °C. The subsequent steps of preparation were the same as in Example 1.

### Example 3

This catalyst was prepared from 1.5 molar precursor solutions and 1.65 molar (15%) precipitating agent sodium carbonate solution using sodium aluminate and dyadic zinc-aluminum solution with Zn/Al=0.5.

300 ml of dyadic zinc-aluminum solution with Zn/Al=0.5 and 1.5 molar in aluminum nitrate was prepared using solid sodium aluminate according to the following procedure:
28.9 g sodium aluminate was dissolved in 208 g water and to which 128.3 g 56% nitric acid was added. During addition of the acid, first, a gel-like aluminum hydroxide precipitate was formed which was dissolved by gradual addition of the acid.

After complete addition of acid to the solution, 12.2 g zinc oxide was added to the solution and stirred for 30 min at 50 °C until zinc oxide was completely dissolved. In this way, 300 ml dyadic zinc-aluminum solution nearly 1.5 M in aluminum nitrate was prepared being designated as solution G.

The solutions E, F were prepared again and the three solutions G, E and F were mixed and warmed up to 55 °C. The 1.65 molar (15 wt%) sodium carbonate solution was also heated in another vessel to the same temperature. Then, both solutions were poured into the precipitation reactor to commence co-precipitation which was performed at a pH of 6.3 to 6.5 and a temperature of 52 to 57 °C. The subsequent steps of preparation were the same as in Example 1.

### Example 4

This catalyst was prepared from 1.70 molar precursor solutions and 1.65 molar (15%) precipitating agent sodium carbonate solution and dyadic zinc-aluminum solution with Zn/Al=0.5.

300 ml of dyadic zinc-aluminum solution with Zn/Al=0.5 and 1.7 molar in aluminum nitrate was prepared according to the following procedure:
12.2 g zinc oxide powder and 127.5 g aluminum nitrate nonahydrate crystals were mixed and the resulting mixture was dissolved in 258 g water at 45 to 50 °C and stirred for 30 min. The solution was labeled as solution H.
500 ml 1.7 M zinc nitrate solution was prepared by dissolving 69.2 g ZnO in 364 g water and 191.3 g 56% nitric acid (solution I).
1200 ml 1.7 M copper nitrate solution was prepared by dissolving 225.5 g CuCO₃.Cu(OH)₂ in 906 g water and 459.1 g 56% nitric acid (solution J).

The three solutions H, I and J were mixed and warmed up to 55 °C. The 1.65 molar (15 wt%) sodium carbonate solution was also heated in another vessel to the same temperature. Then, both solutions were poured into the precipitation reactor to commence co-precipitation which was performed at a pH of 6.3 to 6.5 and a temperature of 52 to 57 °C. The subsequent steps of preparation were the same as in Example 1.

### Example 5

This catalyst was prepared from 1.5 molar precursor solutions and 1.65 molar (15%) precipitating agent sodium carbonate solution and dyadic zinc-aluminum solution with Zn/Al=1.0.

300 ml of dyadic zinc-aluminum solution with Zn/Al=1.0 and 1.5 molar in aluminum nitrate was prepared according to the following procedure:
24.4 g zinc oxide powder and 112.6 g aluminum nitrate nonahydrate crystals were mixed and the resulting mixture was dissolved in 366.3 g water at 45 to 50 °C and stirred for 30 min. The solution was labeled as solution K.
400 ml 1.5 M zinc nitrate solution was prepared by dissolving 48.8 g ZnO in 305.8 g water and 135.0 g 56% nitric acid (solution L).

Solution F was prepared again and the three solutions K, L and F were mixed and warmed up to 55 °C. The 1.65 molar (15 wt%) sodium carbonate solution was also heated in another vessel to the same temperature. Then, both solutions were poured into the precipitation reactor to commence co-precipitation which was performed at a pH of 6.3 to 6.5 and a temperature of 52 to 57 °C. The subsequent steps of preparation were the same as in Example 1.

### Example 6

This catalyst was prepared from 1.5 molar precursor solutions and 1.65 molar (15%) precipitating agent sodium carbonate solution using sodium aluminate and dyadic zinc-aluminum solution with Zn/Al=1.0.

400 ml of dyadic zinc-aluminum solution with Zn/Al=1.0 and 1.5 molar in aluminum nitrate was prepared using solid sodium aluminate according to the following procedure:
28.9 g sodium aluminate was dissolved in 321 g water and to which 128.3 g 56% nitric acid was added. During addition of the acid, first, a gel-like aluminum hydroxide precipitate was formed which was dissolved by gradual addition of the acid.

After complete addition of acid to the solution, 24.4 g zinc oxide was added to the solution and stirred for 30 min at 50 °C until zinc oxide was completely dissolved. In this way, 400 ml dyadic zinc-aluminum solution nearly 1.5 M in aluminum nitrate was prepared being designated as solution M.

The solutions F, L were prepared again and the three solutions F, L and M were mixed and warmed up to 55 °C. The 1.65 molar (15 wt%) sodium carbonate solution was also heated in another vessel to the same temperature. Then, both solutions were poured into the precipitation reactor to commence co-precipitation which was performed at a pH of 6.3 to 6.5 and a temperature of 52 to 57 °C. The subsequent steps of preparation were the same as in Example 1.

### Comparative examples

### Comparative Example 1

This catalyst was made according to the following recipe with 1 molar precursor metal solutions and 1.1 molar (10 wt%) sodium carbonate precipitating agent as a reference or control for comparison with other catalysts.

200 ml 1 M aluminum nitrate solution was prepared by the following method. 75.0 g aluminum nitrate nonahydrate crystals were dissolved in 155 g water at 45 to 50 °C. The solution was labeled as N.

600 ml 1 M zinc nitrate solution was prepared by dissolving 48.8 g ZnO in 501 g water and 135 g of 56% nitric acid (solution O).

Solution C was prepared again and the three solutions N, O and C were mixed and heated to 55 °C. The 1.1 M (15 wt%) sodium carbonate solution was also heated to 55 °C. Precipitation was performed in the precipitation reactor at a pH of 6.3 to 6.5 and a temperature of 52 to 57 °C. The subsequent steps of preparation were the same as in Example 1.

### Comparative Example 2

This catalyst was made according to the following recipe with 1.5 molar metallic precursor solutions and 1.65 molar (15 wt%) sodium carbonate precipitating agent.

200 ml of 1.5 M aluminum nitrate solution was prepared by dissolving 113 g aluminum nitrate nonahydrate crystals in 139 g water at a temperature of 45 to 50 °C. The solution was labeled as solution P.

600 ml 1.5 M zinc nitrate solution was prepared by dissolving 73 g ZnO in 459 g water and 203 g 56% nitric acid (solution Q).

1200 ml 1.5 M copper nitrate solution was prepared by dissolving 199 g CuCO₃.Cu(OH)₂ in 994g water and 405 g 56% HNO₃ (solution F).

The three solutions P, Q and F were mixed and heated to 55 °C. The 1.65 M (15%) sodium carbonate solution was also heated to 55 °C. Precipitation was performed at a pH of 6.3 to 6.5 and a temperature of 52 to 57 °C. The subsequent steps of preparation were the same as in Example 1.

### Comparative Example 3

This catalyst was made by using sodium aluminate as aluminum source with 1.5 molar metal precursor solutions and 1.65 molar (15 wt%) sodium carbonate precipitating agent.

200 ml 1.5 M aluminum nitrate was prepared using solid sodium aluminate (containing 52.2% alumina) using the following recipe:
28.9 g sodium aluminate was dissolved in 94.5 g water to which 128.3 g 56% nitric acid was added. During nitric acid addition, first, a gel of aluminum hydroxide precipitate was formed being dissolved by gradual addition of the acid to completion. The solution was labeled as solution R.

Solutions Q and F were prepared again and the three solutions R, Q and F were mixed together and heated to 55 °C. Precipitation was performed by dropping the mixed solution and the precipitating 1.65 M (15 wt%) sodium carbonate solution heated to 55 °C into the precipitation reactor at a pH of 6.3 to 6.5 and a temperature of 52to 57 °C. The subsequent steps of preparation were the same as in Example 1.

### Comparative Example 4

This catalyst was prepared from 1.70 molar precursor metal solutions and 1.65 molar (15%) sodium carbonate precipitating agent solution.

200 ml 1.7 M aluminum nitrate solution was prepared according to the following recipe:
127.5 g aluminum nitrate nonahydrate crystals were dissolved in 138 g water at a temperature of 45 to 50 °C. The solution was labeled as solution S.
600 ml 1.7 M zinc nitrate solution was prepared by dissolving 83.0 g ZnO in 436 g water and 229.5 g 56% nitric acid (solution T).

Solution J was prepared again and the three solutions S, T and J were mixed and heated to 55 °C. The 1.65 M (15%) sodium carbonate solution was also heated to 55 °C. The precipitation was performed at a pH of 6.3 to 6.5 and a temperature of 52 to 57 °C. The subsequent steps of preparation were the same as in Example 1.

### Comparative Example 5

This catalyst was prepared from 1.85 molar precursor metal solutions and 1.65 molar (15%) sodium carbonate precipitating agent solution.

200 ml 1.85 M aluminum nitrate solution was prepared according to the following recipe:
139.0 g aluminum nitrate nonahydrate crystals were dissolved in 129 g water at a temperature of 45 to 50 °C. The solution was labeled as solution U.
600 ml 1.85 M zinc nitrate solution was prepared by dissolving 90.0 g ZnO in 414 g water and 250 g 56% nitric acid (solution V).
1200 ml 1.85 M copper nitrate was prepared by dissolving 245 g CuCO₃.Cu(OH)₂ in 860 g water and 500 g 56% HNO₃ (solution W).

The three solutions U, V and W were mixed and heated to 55 °C. The 1.65 M (15%) sodium carbonate solution was also heated to 55 °C. The precipitation was performed at a pH of 6.3 to 6.5 and a temperature of 52 to 57 °C. The subsequent steps of preparation were the same as in Example 1.

### Comparative Example 6

This catalyst was prepared from 2.2 molar precursor metal solutions and 1.65 molar (15%) sodium carbonate precipitating agent solution.

200 ml 2.2 M aluminum nitrate solution was prepared according to the following recipe:
165.0 g aluminum nitrate nonahydrate crystals were dissolved in 113 g water at a temperature of 45 to 50 °C. The solution was labeled as solution X.
600 ml 2.2 M zinc nitrate solution was prepared by dissolving 107 g ZnO in 374 g water and 297 g 56% nitric acid (solution Y).
1200 ml 2.2 M copper nitrate was prepared by dissolving 292 g CuCO₃.Cu(OH)₂ in 802 g water and 594 g 56% HNO₃ (solution W).

The three solutions X, Y and Z were mixed and heated to 55 °C. The 1.65 M (15%) sodium carbonate solution was also heated to 55 °C. The precipitation was performed at a pH of 6.3 to 6.5 and a temperature of 52 to 57 °C. The subsequent steps of preparation were the same as in Example 1. Figure 1 shows TEM images of nanoparticles of the Example 5 dyadic Al-Zn solution for different localities and magnifications. The Cu particles could be trapped in 3D morphology of the Al-Zn network.

Figure 1 shows that making a dyadic colloidal solution according to this invention causes the formation of three-dimensional networks comprising zinc and aluminum oxides in which the copper crystallites could be trapped. This situation causes the inhibition of sintering of copper crystallites during catalyst preparation, reduction and also through methanol production because the Cu-crystallites are located in the concavity of energy. Therefore, the activation energy for the sintering process increases and the rate of catalyst deactivation will decrease.

Table 1 shows starting solutions properties, XRD data, textural properties, metal dispersion and the result of catalytic performance tests for Examples and Comparative Examples. The surface area was measured by nitrogen adsorption at 77 K using a *NOVA Quantachrome 2200* High-Speed Gas Sorption Analyzer, version 7.11. The BET surface area was calculated from the multi-point adsorption data in the relative pressure interval from 0.05 to 0.25. The copper surface area was calculated using nitrous oxide chemisorption method using a *BELCAT-A* catalyst analyzer apparatus, according to 2Cu₍ₛ₎ + N₂O_{(g)}→ Cu₂O + N_{2(g)} assuming a value of 1.46×10¹⁹ Cu surface atoms/m² for the copper surface atom density. The crystallite size of Cu was also calculated from the specific surface area of copper with a spherical particle model. Temperature programmed reduction (TPR) measurements of catalysts samples were carried out in a continuous flow apparatus using a U shape quartz micro-reactor fed with a 6.0% H₂ in Ar mixture at 40 ml/min and heating rate of 5 K/min. The performance of the catalyst of the examples for methanol synthesis was determined in a high pressure tubular fixed-bed reactor at 5.0 MPa. The feed gas consisted of CO:CO₂:H₂ = 12:8:80 vol% and GHSV of 17,250 h⁻¹. The by-product concentrations were measured to determine the selectivity to methanol. The by-products are ethanol, higher alcohols (C₂ to C₅), DME, methane, ethane, ethylene, higher hydrocarbons (C₃ to C₈), ethers, esters and ketones, which were measured by a gas chromatograph.

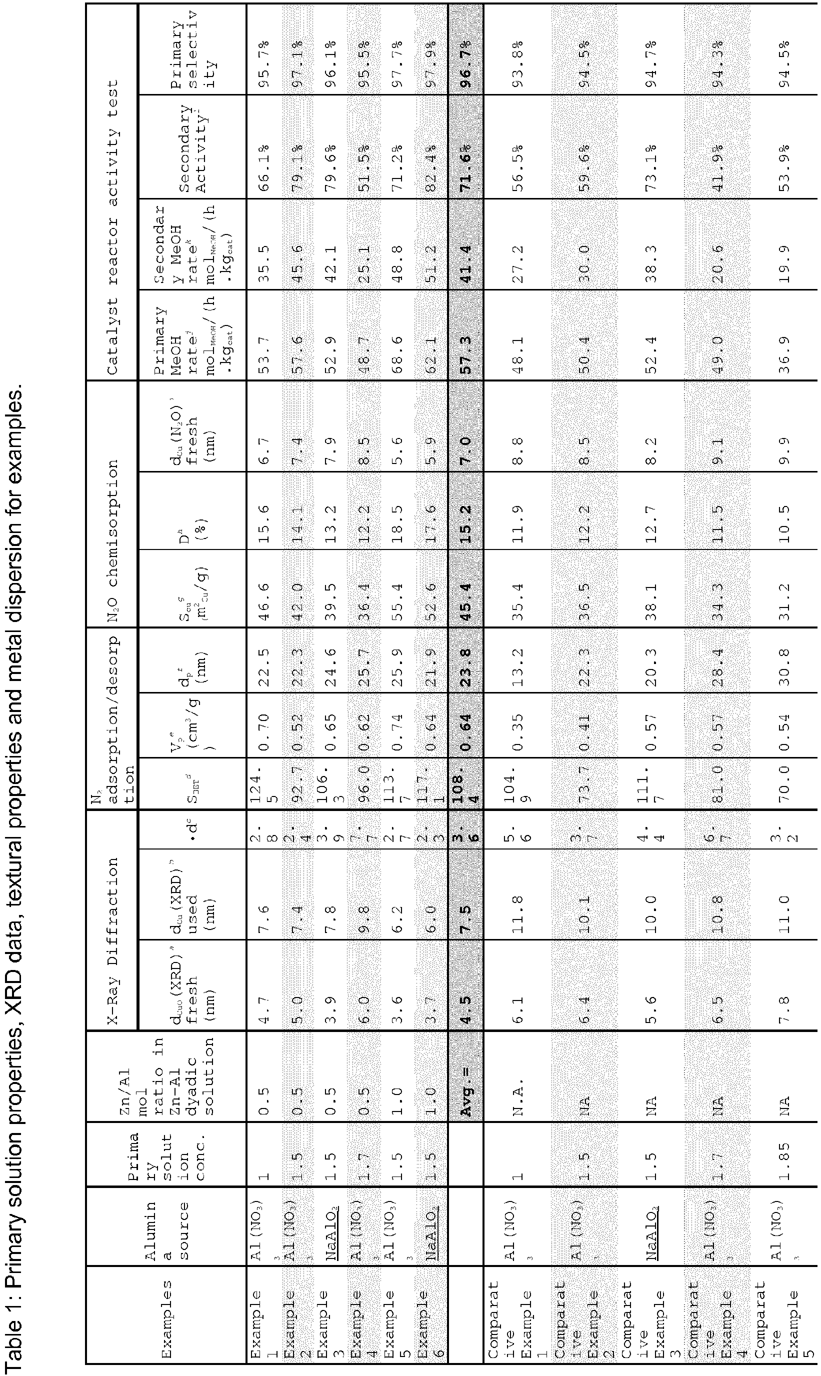

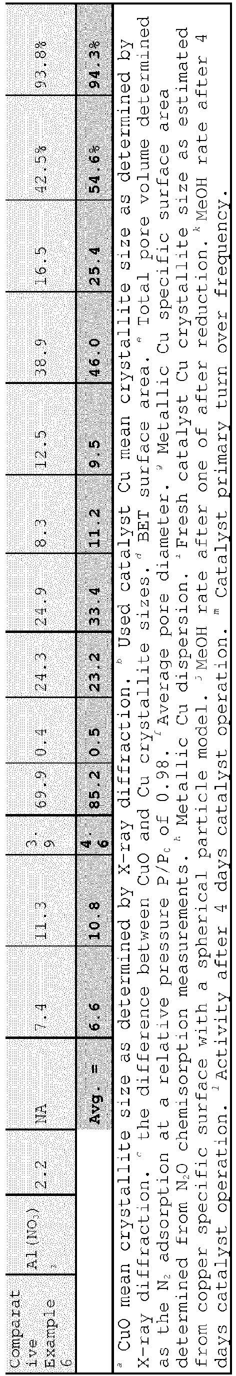

Table 1 shows that upon using dyadic colloidal solution, catalyst BET surface area, active copper surface and catalytic activity increase. Furthermore, the stability or long term activity of the catalysts prepared according to this invention is better than that of comparative example catalysts. The size of the copper crystallites of the catalysts according to Examples 1 to 6 and Comparative Examples 1 to 6 for fresh and used catalysts, during methanol synthesis, were also determined from the XRD patterns by using the Scherrer equation and summarized in Table 1. It shows for the catalysts obtained based on this invention the Cu-crystallite sizes are smaller than the comparative catalysts.

Furthermore, the selectivity of the catalysts prepared based on the invention is significantly higher than that of the prior art catalysts. The by-products being produced in ppm range cause some problem to the process and need an expensive distillation processing. Some by-products such as MEK are not easily removable due to azeotrope formation.

## Claims

1. Method for the preparation of a Cu/Zn/Al catalyst for methanol synthesis comprising the steps of:
(i) Preparing an aqueous colloidal solution by dissolving at least one water soluble or acid soluble Zn(II) compound in an aqueous solution comprising at least one Al(III) salt;
(ii) Preparing an aqueous solution comprising at least one water soluble or acid soluble Cu(II) salt;
(iii) Preparing an aqueous solution comprising at least one water soluble or acid soluble Zn(II) compound;
(iv) Preparing an aqueous solution comprising at least one carbonate salt;
(v) Combining the aqueous solutions of steps (i), (ii), and (iii);
(vi) Combining the aqueous solutions of steps (iv) and (v);
(vii) Precipitating a solid by keeping the solution of step (vi) at a pH value of 6.0 to 8.0 and at a temperature of 35 °C to 70 °C for a period of time of 0.15 to 2 hours;
(viii) Ageing the precipitated solid of step (vii) by keeping the suspension of step (vii) at a pH value of 6.0 to 8.0 and at a temperature of 35 °C to 70 °C for a period of time of 0.15 to 2 hours;
(ix) Filtering the precipitated solid of step (viii);
(x) Optionally washing the filtered solid of step (ix);
(xi) Optionally drying the filtered solid of step (ix) or the washed solid of step (x);
(xii) Calcining the filtered solid of step (ix) or the washed solid of step (x) or the dried solid of step (xi);
(xiii) Optionally tableting the calcined solid of step (xii).

2. The method of claim 1, wherein step (xii) is performed at a temperature of 300 to 400 °C under air flow for a period of time of at least 4 h.

3. The method of claim 1 or 2, wherein step (xiii) is performed by addition of up to 2 wt% graphite based on the calcined solid.

4. The method of any of claims 1 to 3, wherein the Cu(II) salt of step (ii) is copper nitrate or copper carbonate or a combination thereof.

5. The method of any of claims 1 to 4, wherein the Zn(II) compound of step (iii) is zinc nitrate or zinc oxide or a combination thereof.

6. The method of any of claims 1 to 5, wherein the carbonate salt of step (iv) is sodium carbonate or potassium carbonate or a combination thereof.

7. The method of any of claims 1 to 6, wherein steps (vii) and (viii) are performed in two separate reactors having different sizes, and wherein the retention times are 0.1 to 1.0 hour in the first reactor and 0.2 to 2.0 hours in the second reactor.

8. The method of any of claims 1 to 7, wherein the ratio of Zn(II) to Al(III) in the solution of step (i) is 0.2 to 1.3 by mol.

9. The method of any of claims 1 to 8, wherein the combined solution in step (v) comprises Cu(II) in an amount of 0.2 to 3.0 mol/l, Zn(II) in an amount of 0.1 to 2.0 mol/l and Al(III) in an amount of 0.1 to 1.0 mol/l.

10. A Cu/Zn/Al catalyst for methanol synthesis obtained or obtainable by the method of any of claims 1 to 9.

11. A Cu/Zn/Al catalyst for methanol synthesis, preferably obtained or obtainable by the method of any of claims 1 to 9, comprising elemental copper, copper oxides, zinc oxides and aluminum oxides, wherein the surface area of elemental copper as measured by N₂O chemisorption is at least 40 m²/g catalyst, preferably at least 45 m²/g catalyst, more preferably at least 55 m²/g catalyst.

12. The catalyst of claim 11, wherein the copper oxides form particles having a diameter in the range of 2 to 12 nm, preferably in the range of 3 to 7 nm, as measured by XRD.

13. The catalyst of claim 11 or claim 12, wherein the elemental copper forms particles having a diameter in the range of 4 to 15 nm, preferably in the range of 5 to 9 nm, as calculated based on by N₂O chemisorption.

14. The catalyst of any of claims 11 to 13, wherein
(i) the BET surface area as determined by N₂ adsorption is at least 100 m²/g catalyst, preferably at least 120 m²/g catalyst;
(ii) the total pore volume as determined by N₂ adsorption at a relative pressure P/Po of 0.98 is at least 0.45 cm³/g catalyst, preferably at least 0.55 cm³/g catalyst, more preferably at least 0.65 cm³/g catalyst; and
(iii) the average pore diameter is in the range of 10 to 35 nm, preferably in the range of 15 to 27 nm.

15. Use of a catalyst of any of claims 10 to 14 for the synthesis of methanol.
